# EUROPEAN PATENT APPLICATION

(11) **EP 4 624 443 A1**
(43) Date of publication of application: **01.10.2025**
(21) Application number: 22966197.0
(22) Date of filing: 24.11.2022
(51) Int. Cl.: C07C 2/86, C07C 4/06, C07C 1/20, C07C 11/04, B01J 8/26

(54) **NAPHTHA AND METHANOL BASED AROMATIC HYDROCARBON PREPARATION AND OLEFIN CO-PRODUCTION FLUIDIZED BED DEVICE AND METHOD**

(71) Applicant: Dalian Institute Of Chemical Physics, Chinese Academy Of Sciences, Liaoning 116023 (CN); China Shenhua Coal to Liquid and Chemical Co., Ltd, Beijing 100011 (CN)
(72) Inventor: YE, Mao, Dalian, Liaoning 116023 (CN); ZHANG, Tao, Dalian, Liaoning 116023 (CN); ZHANG, Jiming, Beijing 100011 (CN); LIU, Zhongmin, Dalian, Liaoning 116023 (CN); ZHANG, Jinling, Dalian, Liaoning 116023 (CN); YAN, Guochun, Beijing 100011 (CN); TANG, Hailong, Dalian, Liaoning 116023 (CN); WEN, Liang, Beijing 100011 (CN); JIA, Jinming, Dalian, Liaoning 116023 (CN); CAO, Bonan, Beijing 100011 (CN); ZHANG, Cheng, Dalian, Liaoning 116023 (CN); ZHANG, Xiulong, Beijing 100011 (CN); WANG, Xiangao, Dalian, Liaoning 116023 (CN); MA, Xiangang, Dalian, Liaoning 116023 (CN); WANG, Jing, Dalian, Liaoning 116023 (CN); GAO, Mingbin, Dalian, Liaoning 116023 (CN); LI, Xue, Dalian, Liaoning 116023 (CN); XU, Shuliang, Dalian, Liaoning 116023 (CN); LI, Hua, Dalian, Liaoning 116023 (CN); LI, Chenggong, Dalian, Liaoning 116023 (CN)
(74) Representative: Bayramoglu et al.
(86) International application number: PCT/CN2022/134156
(87) International publication number: WO 2024/108506

(57) **Abstract**

The present application discloses a fluidized bed device for coupling naphtha and methanol to prepare aromatics and co-produce olefins and its application method. By using the device, under the action of a catalyst, naphtha reacts with methanol to generate product gas containing aromatics and light olefins as main components. The method in the present application can efficiently and selectively convert linear and branched aliphatic hydrocarbons into aromatics, while also increasing p-xylene production through aromatic methylation reactions, with the p-xylene content in the xylene mixture exceeding 75 wt%. The fluidized bed reactor in the present application achieves increased p-xylene production by controlling the progression of cascade reactions (naphtha→benzene/toluene→p-xylene). Additionally, it utilizes the methylation reaction of benzene/toluene with methanol to provide in-situ heat for the coupled naphtha-methanol aromatization process, thereby achieving autothermal balance.

## Description

### TECHNICAL FIELD

The present application relates to a fluidized bed device and a method for use thereof, and specifically relates to a device for coupling naphtha and methanol to prepare aromatics and co-produce olefins and its application method, belonging to the technical field of chemical engineering.

### BACKGROUND

Aromatics (benzene, toluene, and xylene, collectively referred to as BTX) are important organic chemical raw materials. Among them, para-xylene (PX) is the most noteworthy product in aromatics, mainly used to produce terephthalic acid (PTA), polyethylene terephthalate (PET), polybutylene terephthalate (PBT), and polytrimethylene terephthalate (PTT). In recent years, China's production and consumption of PX have continued to grow. In 2021, China's total PX imports amounted to approximately 13.65 million tons, with an external dependency of about 38%.

Naphtha catalytic reforming technology is the primary technical route for producing aromatics. The composition of naphtha is highly complex, as it serves not only as the main feedstock for catalytic reforming but also as a key raw material for ethylene production via cracking. The composition of naphtha plays a decisive role in the economic efficiency of the device. Generally, feedstock with high aromatic potential content and a suitable distillation range is favorable for catalytic reforming, whereas feedstock with high linear and branched aliphatic hydrocarbon content and low naphthene and aromatic content is suitable for ethylene cracking. To fully utilize naphtha resources and improve economic efficiency, it is necessary to separate linear and branched aliphatic hydrocarbons from naphthenes and aromatics in naphtha, with the former used as feedstock for ethylene production and the latter as feedstock for catalytic reforming devices.

Naphtha fractions have a wide distillation range, making it difficult for conventional separation methods to efficiently separate linear and branched aliphatic hydrocarbons from naphthenes and aromatics. Additionally, catalytic reforming technology struggles to convert linear and branched aliphatic hydrocarbons into aromatics. Naphtha feedstock for catalytic reforming typically requires distillation to remove light fractions (boiling below 60°C), thereby improving the aromatic potential content of the catalytic reforming feedstock. However, fractions with boiling points above 60°C still contain significant amounts of linear and branched aliphatic hydrocarbons that are difficult to convert into aromatics. Therefore, the high-selectivity conversion of linear and branched aliphatic hydrocarbons into aromatics has been a key focus and challenge in the development of naphtha-to-aromatics technology.

Due to thermodynamic equilibrium limitations, para-xylene accounts for only about 24% of the xylene mixture produced by naphtha catalytic reforming devices, necessitating further para-xylene production through isomerization-separation processes. Thus, increasing the para-xylene content in the xylene mixture is an important approach to reducing energy consumption in para-xylene production.

### SUMMARY

The naphtha molecule contains only a small amount of methyl groups (methyl/benzene ring = about 1.3 (molar ratio)). Its molecular structure determines that catalytic reforming/aromatics complex units inevitably produce large amounts of benzene as byproducts.

Methanol aromatization is an emerging process for producing aromatics. However, compared to aromatics, methanol molecules contain excess hydrogen atoms. Therefore, methanol-to-aromatics conversion inevitably yields large amounts of alkanes and hydrogen as byproducts. From the perspective of molecular structure and reaction mechanisms, methanol can provide methyl groups to aromatics, thereby increasing toluene and xylene production. This offers a new technical pathway for coupled aromatics production from naphtha and methanol.

To achieve aromatics production using naphtha and methanol as feedstocks, this application provides a device for coupling naphtha and methanol to prepare aromatics and co-produce olefins and its application method.

According to one aspect of the present application, a fluidized bed device for coupling naphtha and methanol to prepare aromatics and co-produce olefins is provided, comprising a naphtha and methanol coupled aromatization reactor, a regenerator, and a light hydrocarbon aromatization reactor.

The fluidized bed device for coupling naphtha and methanol to prepare aromatics and co-produce olefins comprises a naphtha and methanol coupled aromatization reactor, a regenerator, and a light hydrocarbon aromatization reactor;
the naphtha and methanol coupled aromatization reactor is connected to the regenerator via a spent catalyst delivery pipe I; the regenerator is connected to the naphtha and methanol coupled aromatization reactor via a regenerated catalyst delivery pipe;
a naphtha and methanol coupled aromatization reactor distributor is provided in the naphtha and methanol coupled aromatization reaction zone; the naphtha and methanol coupled aromatization reactor distributor comprises n sub-distributors, sequentially arranged from bottom to top as the 1^{st} sub-distributor to the n^{th} sub-distributor, where 2 ≤ n ≤ 10; the 1st sub-distributor is used to introduce naphtha feedstock; the 2^{nd} to n^{th} sub-distributors are used to introduce methanol feedstock;
the light hydrocarbon aromatization reactor comprises a riser reactor, and the riser reactor is connected to a bed reactor;
the regenerator is connected to the riser reactor via a regenerated catalyst slide valve II; the bed reactor is connected to the regenerator via a spent catalyst delivery pipe II.

Optionally, the upper part of the naphtha and methanol coupled aromatization reactor is provided with a gas-solid separation zone; the gas-solid separation zone is provided with a product gas delivery pipe I; the lower part of the naphtha and methanol coupled aromatization reactor is provided with a naphtha and methanol coupled aromatization reaction zone;
the naphtha and methanol coupled aromatization reactor shell is further provided with a gas-solid separation unit I, a gas-solid separation unit II, and a gas collection chamber I; the gas collection chamber I is located at the top of the gas-solid separation zone, and the gas collection chamber I is connected to the product gas delivery pipe I; the gas outlets of the gas-solid separation unit I and the gas-solid separation unit II are connected to the gas collection chamber I;
the catalyst outlet end of the gas-solid separation unit I is located above the opening end of the stripper I inlet pipe; the inlet of the gas-solid separation unit II is connected to the regenerator; the catalyst outlet end of the gas-solid separation unit II is located above the opening end of the stripper I inlet pipe and between the 1^{st} and 2^{nd} sub-distributors.

Optionally, a stripper I is provided below the naphtha and methanol coupled aromatization reaction zone; the naphtha and methanol coupled aromatization reaction zone is connected to the spent catalyst delivery pipe I via the stripper I.

Optionally, the stripper I is connected to the spent catalyst delivery pipe I via a spent catalyst slide valve I.

Optionally, the gas-solid separation unit I employs one or more sets of gas-solid cyclone separators, each set comprising a first-stage gas-solid cyclone separator and a second-stage gas-solid cyclone separator.

Optionally, the gas-solid separation unit II employs one or more sets of gas-solid cyclone separators, each set comprising a first-stage gas-solid cyclone separator and a second-stage gas-solid cyclone separator.

Optionally, the upper part of the regenerator is provided with a regenerator gas-solid separation zone; the regenerator gas-solid separation zone is provided with a flue gas delivery pipe;
the lower part of the regenerator is provided with a regeneration zone; the spent catalyst delivery pipe I outlet and the spent catalyst delivery pipe II deliver a spent catalyst into the regeneration zone; the lower part of the regenerator is provided with a regenerator distributor for introducing a regeneration gas;
the regenerated catalyst delivery pipe transports the regenerated catalyst from the regeneration zone to the naphtha and methanol coupled aromatization reactor; the regenerated catalyst slide valve II delivers the regenerated catalyst to the riser reactor.

Optionally, the regenerator shell is further provided with a regenerator gas-solid separation unit and a regenerator gas collection chamber; the regenerator gas collection chamber is located at the top of the regenerator gas-solid separation zone; the gas outlet of the regenerator gas-solid separation unit is connected to the regenerator gas collection chamber; the regenerator gas collection chamber is connected to the flue gas delivery pipe.

Optionally, a regenerator stripper is provided below the regeneration zone; the regeneration zone is connected to the regenerated catalyst slide valve I and the regenerated catalyst slide valve II via the regenerator stripper.

Optionally, the regenerated catalyst slide valve I is connected to the naphtha-to-aromatics reactor via the regenerated catalyst delivery pipe; the regenerated catalyst slide valve II is connected to the riser reactor.

Optionally, the regenerator gas-solid separation unit employs one or more sets of gas-solid cyclone separators, each set comprising a first-stage gas-solid cyclone separator and a second-stage gas-solid cyclone separator.

Optionally, the upper part of the bed reactor is provided with a bed reactor gas-solid separation zone; the bed reactor gas-solid separation zone is provided with a product gas delivery pipe II;
the lower part of the bed reactor is provided with a light hydrocarbon aromatization reaction zone; the lower inner part of the light hydrocarbon aromatization reaction zone is provided with a bed reactor distributor; the bed reactor distributor is used to introduce a bed reactor feedstock; the upper end of the riser reactor penetrates the bottom of the bed reactor and is axially inserted into the bed reactor;
the regenerated catalyst slide valve II delivers the catalyst to the feedstock inlet end of the riser reactor.

Optionally, the gas-solid separation zone of the bed reactor is provided with a gas-solid separation unit III and a gas collection chamber II; the gas outlet of the gas-solid separation unit III is connected to the gas collection chamber II; the catalyst outlet of the gas-solid separation unit III is located in the light hydrocarbon aromatization reaction zone; the gas collection chamber II is connected to the product gas delivery pipe II located outside the bed reactor.

Optionally, the light hydrocarbon aromatization reaction zone is connected to a stripper II, and the bed reactor is connected to the spent catalyst delivery pipe II via the stripper II.

Optionally, the stripper II is connected to the spent catalyst delivery pipe II via a spent catalyst slide valve II.

Optionally, the gas-solid separation unit III is a gas-solid cyclone separator; the catalyst outlet of the gas-solid separation unit III is located above the outlet end of the riser reactor.

The present application also provides a method for coupling naphtha and methanol to prepare aromatics and co-produce olefins, the device according to any one of claims 1 to 4 is used; the catalyst is a metal molecular sieve bifunctional catalyst;
a feedstock containing naphtha is introduced into the naphtha and methanol coupled aromatization reactor via the 1^{st} sub-distributor; a feedstock containing methanol is introduced into the naphtha and methanol coupled aromatization reactor via the 2^{nd} to n^{th} sub-distributors; a riser reactor feedstock containing light alkanes is introduced into the riser reactor; a bed reactor feedstock is introduced into the bed reactor; a regeneration gas is introduced into the regenerator;
the naphtha and methanol coupled aromatization reactor and the bed reactor output a product gas, and a spent catalyst is delivered to the regenerator via the spent catalyst delivery pipe I and the spent catalyst delivery pipe II; after the spent catalyst is regenerated by reacting with regeneration gas in the regenerator, it is delivered to the naphtha and methanol coupled aromatization reactor and the riser reactor; the regenerator discharges a flue gas.

Optionally, the catalyst is a metal-modified HZSM-5 zeolite molecular sieve; a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

Optionally, the naphtha is at least one selected from the group consisting of coal direct liquefaction naphtha, coal indirect liquefaction naphtha, straight-run naphtha, and hydrocracking naphtha.

Optionally, the feedstock containing naphtha further comprises unconverted naphtha separated from the product gas flow, and the unconverted naphtha comprises linear aliphatic hydrocarbons, branched aliphatic hydrocarbons, and naphthenes of C₄-C₁₂.

Optionally, the reaction conditions of the naphtha and methanol coupled aromatization reaction zone are: gas superficial linear velocity of 0.5-2.0 m/s, reaction temperature of 500-600°C, reaction pressure of 100-500 kPa, and bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among 500°C, 520°C, 530°C, 540°C, 550°C, 560°C, 570°C, 580°C, 590°C, 600°C or any range between two values.

Optionally, the reaction pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Preferably, the carbon content in the regenerated catalyst is ≤0.5 wt%.

Preferably, the regeneration gas is at least one selected from the group consisting of oxygen, air, and oxygen-enriched air.

Preferably, the reaction conditions of the regeneration zone are: gas superficial linear velocity of 0.5-2.0 m/s, regeneration temperature of 600-750°C, regeneration pressure of 100-500 kPa, and bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the regeneration temperature is independently selected from any value among 600°C, 615°C, 630°C, 645°C, 670°C, 685°C, 700°C, 715°C, 730°C, 745°C, 750°C or any range between two values.

Optionally, the regeneration pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Optionally, the riser reactor feedstock further comprises water vapor, with a water vapor content of 0-80 wt%.

Optionally, the light alkanes in the riser reactor feedstock are obtained by separation from the product gas flow.

Optionally, the reaction conditions of the riser reactor are: gas superficial linear velocity of 3.0-10.0 m/s, temperature of 580-700°C, pressure of 100-500 kPa, and bed density of 50-150 kg/m³.

Optionally, the gas superficial velocity is independently selected from any value among 3.0m/s, 3.5m/s, 4.0m/s, 4.5m/s, 5.0m/s, 5.5m/s, 6.0m/s, 6.5m/s, 7.0m/s, 7.5m/s, 8.0m/s, 8.5m/s, 9.0m/s, 9.5m/s, 10.0m/s or any range between two values.

Optionally, the temperature is independently selected from any value among 580°C, 590°C, 600°C, 610°C, 620°C, 630°C, 640°C, 650°C, 660°C, 670°C, 680°C, 690°C, 700°C or any range between two values.

Optionally, the pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 50kg/m³, 60kg/m³, 70kg/m³, 80kg/m³, 90kg/m³, 100kg/m³, 110kg/m³, 120kg/m³, 130kg/m³, 140kg/m³, 150kg/m³ or any range between two values.

Optionally, the bed reactor feedstock is obtained by separation from the product gas flow.

Optionally, the bed reactor feedstock comprises unconverted naphtha separated from the product gas flow, and the unconverted naphtha comprises linear aliphatic hydrocarbons, branched aliphatic hydrocarbons, and naphthenes of C₄-C₁₂.

Optionally, the bed reactor feedstock comprises C₃, C₄, and C₅ hydrocarbons.

Optionally, the bed reactor feedstock comprises C₄ and C₅ hydrocarbons.

Optionally, the reaction conditions of the light hydrocarbon aromatization reaction zone are: gas superficial linear velocity of 0.5-2.0 m/s, reaction temperature of 550-665°C, reaction pressure of 100-500 kPa, and bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among550°C, 560°C, 570°C, 580°C, 590°C, 600°C, 610°C, 620°C, 630°C, 640°C, 650°C, 660°C, 665°C or any range between two values.

Optionally, the reaction pressure is independently selected from any value among100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Optionally, naphtha enters the naphtha and methanol coupled aromatization reaction zone via the 1^{st} sub-distributor of the naphtha and methanol coupled aromatization reactor distributor, contacts the catalyst from the regenerator, and generates a product gas flow containing BTX, light olefins, hydrogen, light alkanes, combustible gas, heavy aromatics, and unconverted naphtha; the catalyst from the regenerator enters the gas-solid separation unit II to achieve gas-solid separation; a degassed catalyst enters the space between the 1^{st} and 2^{nd} sub-distributors; methanol enters the naphtha and methanol coupled aromatization reaction zone via the 2^{nd} to n^{th} sub-distributors of the naphtha and methanol coupled aromatization reactor distributor, reacts with benzene and toluene in the product gas flow to undergo methylation, generating para-xylene; the catalyst from the regenerator becomes spent catalyst due to coking in the naphtha and methanol coupled aromatization reaction zone; the product gas flow enters the gas-solid separation unit I to remove the spent catalyst entrained in it, then enters the gas collection chamber I, and is delivered to downstream sections via the product gas delivery pipe I; the spent catalyst in the naphtha and methanol coupled aromatization reaction zone enters the stripper I via the opening end of the stripper I inlet pipe, undergoes stripping, and after stripping, passes through the spent catalyst slide valve I and the spent catalyst delivery pipe I to enter downstream sections;
the regeneration gas is introduced into the regeneration zone of the regenerator via the regenerator distributor, contacts the spent catalyst from the naphtha and methanol coupled aromatization reactor and the spent catalyst from the light hydrocarbon aromatization reactor, and the coke on the spent catalyst reacts with the regeneration gas to generate the flue gas, converting the spent catalyst into the regenerated catalyst;
the riser reactor feedstock is introduced into the riser reactor via the inlet end of the riser reactor, contacts and reacts with the regenerated catalyst from the regenerator, and the riser reactor feedstock is converted into a flow containing BTX, light olefins, H₂, and other components under the action of the catalyst, then enters the lower inner part of the light hydrocarbon aromatization reaction zone in the bed reactor via the outlet end of the riser reactor;
the bed reactor feedstock is introduced into the light hydrocarbon aromatization reaction zone via the bed reactor distributor, contacts the catalyst from the riser reactor, and generates a light hydrocarbon aromatization product gas containing BTX, light olefins, H₂, and other components, while the catalyst becomes the spent catalyst.

Optionally, the product gas flow enters the gas-solid separation unit I to remove entrained spent catalyst, then enters the gas collection chamber I, and is conveyed to downstream sections via the product gas delivery pipe I.

The spent catalyst in the naphtha and methanol coupled aromatization reaction enters the stripper I through the open end of the stripper I inlet pipe, undergoes stripping, and is then transported to a downstream section via the spent catalyst slide valve I and the spent catalyst delivery pipe I.

Specifically, the downstream section is the regenerator.

The term "light olefins" refers to ethylene and propylene.

The term "light alkanes" refers to ethane and propane.

The term "combustible gas" includes methane and CO.

The term "heavy aromatics" refers to aromatic hydrocarbons with nine or more carbon atoms per molecule.

Optionally, the method includes: the spent catalyst from the naphtha and methanol coupled aromatization reaction sequentially passes through the stripper I, the spent catalyst slide valve I, and the spent catalyst delivery pipe I into the regenerator, where it contacts and reacts with the regeneration gas to produce flue gas and regenerated catalyst;
Optionally, the method includes: the spent catalyst from the light hydrocarbon aromatization reaction zone sequentially passes through the stripper II, the spent catalyst slide valve II, and the spent catalyst delivery pipe II into the regenerator, where it contacts and reacts with the regeneration gas to produce flue gas and regenerated catalyst;
the flue gas enters the regenerator gas-solid separation unit to remove entrained regenerated catalyst, then enters the regenerator gas collection chamber, and is conveyed to downstream sections via the flue gas delivery pipe.

Optionally, the regenerated catalyst sequentially passes through the regenerator stripper, the regenerated catalyst slide valve I, and the regenerated catalyst delivery pipe into the naphtha and methanol coupled aromatization reactor.

Optionally, the regenerated catalyst sequentially passes through the regenerator stripper and the regenerated catalyst slide valve II into the light hydrocarbon aromatization reactor.

Optionally, the light hydrocarbon aromatization product gas enters the gas-solid separation unit III to remove entrained catalyst, then enters the gas collection chamber II, and is conveyed to downstream sections via the product gas delivery pipe II.

The catalyst in the light hydrocarbon aromatization reaction zone passes through the stripper II, the spent catalyst slide valve II, and the spent catalyst delivery pipe II into downstream sections.

Specifically, the downstream section is the regenerator.

The C₃, C₄ and C₅ hydrocarbons refer to hydrocarbons with 3, 4 and 5 carbon atoms.

The C₄ and C₅ hydrocarbons refer to hydrocarbons with 4 and 5 carbon atoms.

In the present application, the components of the naphtha include C₄-C₁₂ linear aliphatic hydrocarbons, branched aliphatic hydrocarbons, naphthenes, and aromatics.

In the present application, the term "aromatics" refers to benzene, toluene, and xylene, collectively termed BTX.

In the method of the present application, the naphtha feedstock has an aromatics potential content of 0-80 wt% with a single-pass conversion rate of 70-95 wt% and a single-pass conversion rate of methanol is about 100 wt%. Unconverted naphtha is separated from the product gas and recycled as feedstock to the naphtha and methanol coupled aromatization reactor. A portion of light alkanes is separated from the product gas and recycled as feedstock to the riser reactor in the light hydrocarbon aromatization reactor. C₃, C₄, and C₅ hydrocarbons are separated from the product gas and recycled as feedstock to the bed reactor in the light hydrocarbon aromatization reactor. The final product distribution is: 60-72 wt% BTX, 8-15 wt% light olefins, 3-7 wt% hydrogen, 3-7 wt% light alkanes, 4-6 wt% combustible gas, 4-8 wt% heavy aromatics, and 0.5-1 wt% coke. The para-xylene content in the mixed xylene product exceeds 60-75 wt%.

The beneficial effects of the present application include:
(1) The method for preparing aromatics from naphtha and methanol co-producing olefins described in the present application can convert linear and branched aliphatic hydrocarbons into aromatics with high selectivity. Therefore, the method described in the present application has a wide range of raw material adaptability, and aromatics can be prepared using naphtha with a low potential aromatic content as a raw material.
(2) By employing the light hydrocarbon aromatization reactor and a metal molecular sieve bifunctional catalyst, the method achieves aromatization of light alkanes, C₄, and C₅ hydrocarbons, significantly improving the overall aromatics yield in naphtha-to-aromatics technology.
(3) The device for coupling naphtha and methanol to prepare aromatics and co-produce olefins in the present application is equipped with a naphtha and methanol coupled aromatization reactor, which includes multiple sub-distributors and a gas-solid separation unit II. Naphtha enters the naphtha and methanol coupled aromatization reaction zone through the 1^{st} sub-distributor, while methanol enters the naphtha and methanol coupled aromatization reaction zone separately through the 2nd to nth sub-distributors. The gas-solid separation unit II directly delivers highly active catalyst from the regenerator above the 1^{st} sub-distributor. The naphtha and methanol coupled aromatization reactor is a fluidized bed reactor adapted for cascade reactions. Naphtha is first converted into benzene and toluene in the lower part of the naphtha and methanol coupled aromatization reaction zone, then flows upward to the middle and upper parts of the reaction zone, where benzene, toluene and methanol undergo methylation reactions to further produce p-xylene, thereby achieving the purpose of producing the target product p-xylene. The highly active catalyst from the regenerator directly enters the lower part of the naphtha and methanol coupled aromatization reaction zone, which facilitates the conversion of naphtha and methanol coupled aromatization and improves naphtha conversion rate. Methanol directly enters the middle and upper parts of the naphtha and methanol coupled aromatization reaction zone, effectively reducing the residence time of p-xylene in the reaction zone, inhibiting the isomerization reaction of p-xylene, increasing the p-xylene content in xylenes (which can reach 75 wt% under optimal process conditions), while significantly reducing the separation energy consumption of p-xylene. In short, in the naphtha and methanol coupled aromatization reactor, the naphtha feedstock flows from bottom to top. During its conversion to aromatics, by stepwise addition of methylation feedstock (methanol), the process of cascade reactions (naphtha→benzene/toluene→p-xylene) is controlled, achieving increased p-xylene production.
(4) The naphtha aromatization reaction is strongly endothermic, requiring absorption of 1.1-1.6 MJ of heat per kg of naphtha converted to aromatics. The methylation reaction of methanol and aromatics is strongly exothermic, releasing more than 2.0 MJ of heat per kg of methanol converted to methyl groups on aromatics. Therefore, the production of PX from benzene, toluene and methanol can also provide heat in situ for the coupled naphtha and methanol aromatization reaction, achieving autothermal balance.
(5) The naphtha and methanol coupled aromatics and olefins co-production device in this application is equipped with an independent light hydrocarbon aromatization reactor. Light alkanes are very stable and require high reaction temperatures. The temperature of the light hydrocarbon aromatization reactor is higher than that of the naphtha and methanol coupled aromatization reactor. Light alkanes and C₄, C₅ hydrocarbons undergo aromatization reactions in the independent light hydrocarbon aromatization reactor, improving reaction rate and aromatic yield.

### BRIEF DESCRIPTION OF THE DRAWINGS

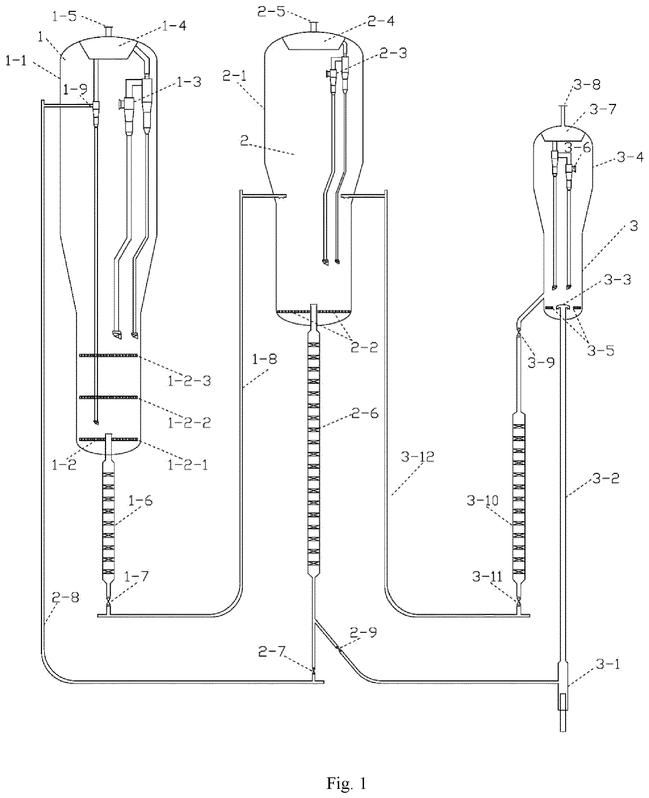

Fig. 1 is a schematic diagram of a device for coupling naphtha and methanol to prepare aromatics and co-produce olefins according to one embodiment of the present application.

### List of Components and Reference Numerals:

1 naphtha and methanol coupled aromatization reactor;
1-1 naphtha and methanol coupled aromatization reactor shell;
1-2 naphtha and methanol coupled aromatization reactor distributor;
1-3 gas-solid separation unit I;
1-4 gas collection chamber I;
1-5 product gas delivery pipe I;
1-6 stripper I;
1-7 spent catalyst slide valve I;
1-8 spent catalyst delivery pipe I;
1-9 gas-solid separation unit II
1-2-1 1^{st} sub-distributor; 1-2-2 2^{nd} sub-distributor; 1-2-3 3^{rd} sub-distributor.
2 regenerator;
2-1 regenerator shell;
2-2 regenerator distributor;
2-3 regenerator gas-solid separation unit;
2-4 regenerator gas collection chamber;
2-5 flue gas delivery pipe;
2-6 regenerator stripper;
2-7 regenerated catalyst slide valve I;
2-8 regenerated catalyst delivery pipe;
2-9 regenerated catalyst slide valve II.
3 light hydrocarbon aromatization reactor;
3-1 inlet end of the riser reactor;
3-2 middle section of the riser reactor;
3-3 outlet end of the riser reactor;
3-4 bed reactor shell;
3-5 bed reactor distributor;
3-6 gas-solid separation unit III;
3-7 gas collection chamber II;
3-8 product gas delivery pipe II;
3-9 stripper II;
3-10 spent catalyst slide valve II;
3-11 spent catalyst delivery pipe II.

### DETAILED DESCRIPTION OF THE EMBODIMENTS

The present application will be described in detail below with reference to examples, but the present application is not limited to these examples.

The following describes possible embodiments.

The present application provides a fluidized bed device for coupling naphtha and methanol to prepare aromatics and co-produce olefins, as shown in Fig. 1. The device comprises a naphtha and methanol coupled aromatization reactor 1, a regenerator 2, and a light hydrocarbon aromatization reactor 3.

The naphtha and methanol coupled aromatization reactor 1 comprises: a naphtha and methanol coupled aromatization reactor shell 1-1, a naphtha and methanol coupled aromatization reactor distributor 1-2, a gas-solid separation unit I 1-3, a gas collection chamber I 1-4, a product gas delivery pipe I 1-5, a stripper I 1-6, a spent catalyst slide valve I 1-7, a spent catalyst delivery pipe I 1-8, and a gas-solid separation unit II 1-9.

The naphtha and methanol coupled aromatization reactor distributor 1-2 comprises: a 1^{st} sub-distributor 1-2-1, a 2^{nd} sub-distributor 1-2-2, and a 3^{rd} sub-distributor 1-2-3.

The naphtha and methanol coupled aromatization reactor shell 1-1 comprises an upper naphtha and methanol coupled aromatization reactor shell and a lower naphtha and methanol coupled aromatization reactor shell. The upper naphtha and methanol coupled aromatization reactor shell encloses a gas-solid separation zone, and the lower naphtha and methanol coupled aromatization reactor shell encloses a naphtha and methanol coupled aromatization reaction zone. The naphtha and methanol coupled aromatization reactor shell is provided with an outlet of the regenerated catalyst delivery pipe 2-8.

The naphtha and methanol coupled aromatization reactor distributor 1-2 is provided in the lower part of the naphtha and methanol coupled aromatization reaction zone. The naphtha and methanol coupled aromatization reactor distributor comprises three sub-distributors, which are sequentially arranged from bottom to top as the 1^{st} sub-distributor 1-2-1 to the 3^{rd} sub-distributor 1-2-3. The 1^{st} sub-distributor is used for introducing naphtha feedstock. The 2^{nd} sub-distributor to the 3rd sub-distributor are used for introducing methanol feedstock.

The naphtha and methanol coupled aromatization reactor shell 1-1 is further provided with the gas-solid separation unit I 1-3, the gas-solid separation unit II 1-9 and the gas collection chamber I 1-4. The gas collection chamber I 1-4 is located at the inner top of the naphtha and methanol coupled aromatization reactor shell. The gas outlet of the gas-solid separation unit I 1-3 is connected to the gas collection chamber I 1-4. The gas collection chamber I 1-4 is connected to the product gas delivery pipe I 1-5. The catalyst outlet end of the gas-solid separation unit I 1-3 is located above the opening end of the inlet pipe of the stripper I 1-6. The inlet of the gas-solid separation unit II 1-9 is connected to the regenerator 2. The gas outlet of the gas-solid separation unit II 1-9 is connected to the gas collection chamber I 1-4. The catalyst outlet end of the gas-solid separation unit II 1-9 is located above the opening end of the inlet pipe of the stripper I 1-6, and between the 1^{st} sub-distributor 1-2-1 and the 2^{nd} sub-distributor 1-2-2.

The stripper I 1-6 is provided beneath the naphtha and methanol coupled aromatization reaction zone. The inlet of the stripper I 1-6 is located inside the naphtha and methanol coupled aromatization reactor shell 1-1. The outlet of the stripper I 1-6 is located outside the naphtha and methanol coupled aromatization reactor shell 1-1 and is connected to the spent catalyst slide valve I 1-7. The opening end of the inlet of the stripper I 1-6 is located above the 1^{st} sub-distributor 1-2-1 of the naphtha-to-aromatics reactor.

The spent catalyst slide valve I 1-7 is provided beneath the stripper I 1-6. The inlet of the spent catalyst slide valve I 1-7 is connected to the outlet of the stripper I 1-6. The outlet of the spent catalyst slide valve I 1-7 is connected to the inlet of the spent catalyst delivery pipe I 1-8. The outlet of the spent catalyst delivery pipe I 1-8 is connected to the regenerator shell 2-1.

The spent catalyst slide valve I 1-7 is used for controlling the circulation amount of spent catalyst.

In a preferred embodiment, the gas-solid separation unit I 1-3 employs one or more sets of gas-solid cyclone separators, each set comprising a first-stage gas-solid cyclone separator and a second-stage gas-solid cyclone separator.

In a preferred embodiment, the gas-solid separation unit II 1-9 employs one or more sets of gas-solid cyclone separators, each set comprising a first-stage gas-solid cyclone separator and a second-stage gas-solid cyclone separator.

The regenerator 2 comprises: the regenerator shell 2-1, the regenerator distributor 2-2, the regenerator gas-solid separation unit 2-3, the regenerator gas collection chamber 2-4, the flue gas delivery pipe 2-5, the regenerator stripper 2-6, the regenerated catalyst slide valve I 2-7, the regenerated catalyst delivery pipe 2-8, and the regenerated catalyst slide valve II 2-9.

The regenerator shell 2-1 comprises the regenerator upper shell and the regenerator lower shell, wherein the regenerator upper shell encloses the gas-solid separation zone, and the regenerator lower shell encloses the regeneration zone; the regenerator shell 2-1 is provided with an outlet of the spent catalyst delivery pipe I 1-8 and the outlet of the spent catalyst delivery pipe II 3-11.

The regenerator distributor 2-2 is provided at the lower part of the regeneration zone, and the regenerator distributor 2-2 is used for introducing the regeneration gas.

The regenerator shell 2-1 is also provided with a regenerator gas-solid separation unit 2-3 and a regenerator gas collection chamber 2-4; the regenerator gas collection chamber 2-4 is located at the inner top of the regenerator shell 2-1; the gas outlet of the regenerator gas-solid separation uint 2-3 is connected to the regenerator gas collection chamber 2-4; the regenerator gas collection chamber 2-4 is connected to the flue gas delivery pipe 2-5; the catalyst outlet end of the regenerator gas-solid separation unit 2-3 is located above the opening end of the inlet pipe of the regenerator stripper 2-6.

The regenerator stripper 2-6 is provided below the regeneration zone; the inlet of the regenerator stripper 2-6 is located inside the regenerator shell 2-1; the outlet of the regenerator stripper 2-6 is located outside the regenerator shell 2-1, and is connected to the regenerated catalyst slide valve I 2-7 and the regenerated catalyst slide valve II 2-9; the opening end of the inlet of the regenerator stripper 2-6 is located above the regenerator distributor 2-2.

The regenerated catalyst slide valve I 2-7 is connected to the inlet of the regenerated catalyst delivery pipe 2-8, and the outlet of the regenerated catalyst delivery pipe 2-8 is connected to the inlet of the gas-solid separation unit II 1-9.

The regenerated catalyst slide valve I 2-7 is used to control the circulation amount of the regenerated catalyst.

The regenerated catalyst slide valve II 2-9 is used to control the circulation amount of the regenerated catalyst.

In a preferred embodiment, the regenerator gas-solid separation unit 2-3 uses one or more groups of gas-solid cyclone separators, and each group of gas-solid cyclone separators includes the first-stage gas-solid cyclone separator and the second-stage gas-solid cyclone separator.

The light hydrocarbon aromatization reactor 3 comprises: the inlet end of the riser reactor 3-1, the middle part of the riser reactor 3-2, the outlet end of the riser reactor 3-3, the bed reactor shell 3-4, the bed reactor distributor 3-5, the gas-solid separation unit III 3-6, the gas collection chamber II 3-7, the product gas delivery pipe II 3-8, the stripper II 3-9, the spent catalyst slide valve II 3-10, and the spent catalyst delivery pipe II 3-11.

The bed reactor shell 3-4 comprises an upper shell of the bed reactor and a lower shell of the bed reactor, the upper shell of the bed reactor encloses a gas-solid separation zone, and the lower shell of the bed reactor encloses a light hydrocarbon aromatization reaction zone; the bed reactor distributor 3-5 is provided at the inner lower part of the light hydrocarbon aromatization reaction zone; the upper section of the riser reactor penetrates the bottom of the bed reactor and is axially inserted in the bed reactor; the outlet end of the riser reactor 3-3 is located at the inner lower part of the light hydrocarbon aromatization reaction zone.

The gas-solid separation zone of the bed reactor is provided with the gas-solid separation unit III 3-6 and a gas collection chamber II 3-7; the gas outlet of the gas-solid separation unit III 3-6 is connected to the gas collection chamber II 3-7; the catalyst outlet of the gas-solid separation unit III 3-6 is located in the light hydrocarbon aromatization reaction zone; the gas collection chamber II 3-7 is connected to the product gas delivery pipe II 3-8 located outside the bed reactor.

The stripper II 3-9 and the spent catalyst slide valve II 3-10 are provided outside the shell of the bed reactor; the inlet of the stripper II 3-9 is located at the lower shell of the bed reactor; the outlet of the stripper II 3-9 is connected to the inlet of the spent catalyst slide valve II 3-10, the outlet of the spent catalyst slide valve II 3-10 is connected to the inlet of the spent catalyst delivery pipe II 3-11, and the outlet of the spent catalyst delivery pipe II 3-11 is connected to the regenerator shell 2-1.

In a preferred embodiment, the gas-solid separation unit III 3-6 is a gas-solid cyclone separator; the catalyst outlet of the gas-solid separation unit III 3-6 is located above the outlet end of the riser reactor 3-3.

In a preferred embodiment, a gas collection chamber II 3-7 is provided at the inner top of the bed reactor.

In a preferred embodiment, the bed reactor distributor 3-5 is used to feed the bed reactor feedstock.

In a preferred embodiment, the inlet end of the riser reactor 3-1 is connected to the regenerated catalyst slide valve II 2-9 through a pipeline.

In a preferred embodiment, the inlet end of the riser reactor 3-1 is used to feed the catalyst and the riser reactor feedstock.
The present application provides a method for coupling naphtha and methanol to prepare aromatics and co-produce olefins, the method using any of the above-mentioned devices and a metal molecular sieve bifunctional catalyst;
Optionally, The catalyst adopts metal-modified HZSM-5 zeolite molecular sieve; the metal used for metal modification is at least one selected from the group consisting of La, Zn, Ga, Fe, Mo, and Cr;
the metal modification method comprises: placing the HZSM-5 zeolite molecular sieve in a metal salt solution, impregnating, drying and calcining to obtain the metal modified HZSM-5 zeolite molecular sieve.

The method comprises the following steps:
Naphtha enters the naphtha and methanol coupled aromatization reaction zone through the 1^{st} sub-distributor 1-2-1 of the naphtha and methanol coupled aromatization reactor distributor 1-2, and contacts with catalyst from the regenerator 2 to generate a product gas flow containing BTX, light olefins, hydrogen, light alkanes, combustible gas, heavy aromatics and unconverted naphtha, while the catalyst; the catalyst from the regenerator 2 enters the gas-solid separation unit II 1-9 to achieve gas-solid separation, then the degassed catalyst enters between the 1^{st} sub-distributor 1-2-1 and the 2nd sub-distributor 1-2-2; methanol enters the naphtha and methanol coupled aromatization reaction zone through the 2^{nd} sub-distributor 1-2-2 to the 3^{rd} sub-distributor 1-2-3 of the naphtha and methanol coupled aromatization reactor distributor 1-2 respectively, and undergoes methylation reaction with benzene and toluene in the product gas flow to generate para-xylene; the catalyst from the regenerator 2 is converted into spent catalyst through coking in the naphtha and methanol coupled aromatization reaction zone; the product gas flow enters the gas-solid separation unit I 1-3 to remove the entrained spent catalyst, then enters the gas collection chamber I 1-4, and is delivered to downstream units through the product gas delivery pipe I 1-5; the spent catalyst in the naphtha and methanol coupled aromatization reaction zone enters the stripper I 1-6 through the opening end of the inlet pipe of the stripper I 1-6 for stripping, and after stripping, passes through the spent catalyst slide valve I 1-7 and the spent catalyst delivery pipe I 1-8 to enter the regenerator 2.
passing the regenerated gas into the regeneration zone of the regenerator 2 through the regenerator distributor 2-2, and contacting with the spent catalyst, the coke on the spent catalyst reacts with the regenerated gas to generate flue gas, and at the same time, the spent catalyst is converted into the regenerated catalyst; the flue gas enters the regenerator gas-solid separation unit 2-3 to remove the regenerated catalyst carried therein, and then enters the regenerator gas collection chamber 2-4, and enters the downstream section through the flue gas delivery pipe 2-5; the regenerated catalyst passes through the regenerator stripper 2-6, the regenerated catalyst slide valve I 2-7 and the regenerated catalyst delivery pipe 2-8 in sequence to enter the naphtha and methanol coupled aromatization reactor 1; the regenerated catalyst passes through the regenerator stripper 2-6 and the regenerated catalyst slide valve II 2-9 in sequence to enter the light hydrocarbon aromatization reactor 3;
feeding the riser reactor feedstock into the riser reactor through the inlet end of the riser reactor 3-1, contacting and reacting with the regenerated catalyst from the regenerator, and converting the riser reactor feedstock into a flow containing components such as BTX, light olefins and H₂ under the action of the catalyst, and then entering the inner lower part of the light hydrocarbon aromatization reaction zone in the bed reactor through the outlet end of the riser reactor 3-3; feeding the bed reactor feedstock into the light hydrocarbon aromatization reaction zone through the bed reactor distributor 3-5, contacting with the catalyst from the riser reactor, and generating a light hydrocarbon aromatization product gas containing components such as BTX, light olefins and H₂; the light hydrocarbon aromatization product gas enters the gas-solid separation unit **III** 3-6 to remove the catalyst carried therein, and then enters the gas collection chamber II 3-7, and then enters the downstream section through the product gas delivery pipe II 3-8; the catalyst in the light hydrocarbon aromatization reaction zone passes through the stripper II 3-9, the spent catalyst slide valve II 3-10 and the spent catalyst delivery pipe II 3-11 to enter the regenerator 2.

The light olefins are ethylene and propylene.

The light alkanes are ethane and propane.

The combustible gas includes methane, CO and the like.

The heavy aromatic hydrocarbons refer to aromatic hydrocarbons having 9 or more carbon atoms in the molecule.

In a preferred embodiment, the naphtha is selected from at least one of coal direct liquefaction naphtha, coal indirect liquefaction naphtha, straight run naphtha and hydrocracked naphtha.

In a preferred embodiment, the naphtha further comprises unconverted naphtha separated from the product gas flow.

In a preferred embodiment, the process conditions of the naphtha and methanol coupled aromatization reaction zone are: gas superficial velocity of 0.5-2.0 m/s, reaction temperature of 500-600 °C, reaction pressure of 100-500 kPa, and bed density of 150-700 kg/m³.

In a preferred embodiment, the carbon content in the regenerated catalyst is ≤0.5 wt%.

In a preferred embodiment, the regeneration gas is selected from at least one of oxygen, air and oxygen enriched air.

In a preferred embodiment, the process conditions in the regeneration zone are: gas superficial linear velocity is 0.5-2.0 m/s, regeneration temperature is 600-750 °C, regeneration pressure is 100-500 kPa, and bed density is 150-700 kg/m³.

In a preferred embodiment, the riser reactor feedstock comprises water vapor and light alkanes separated from the product gas flow.

In a preferred embodiment, the water vapor content in the riser reactor feed is 0-80 wt%.

In a preferred embodiment, the process conditions of the riser reactor are: gas superficial velocity of 3.0-10.0 m/s, temperature of 580-700°C, pressure of 100-500 kPa, and bed density of 50-150 kg/m³.

In a preferred embodiment, the bed reactor feedstock comprises unconverted naphtha separated from the product gas flow, with the main components of the unconverted naphtha being linear aliphatic hydrocarbons, branched aliphatic hydrocarbons and naphthenes of C₄-C₁₂.

In a preferred embodiment, the bed reactor feedstock comprises C₃, C₄ and C₅ hydrocarbons.

In a preferred embodiment, the bed reactor feedstock comprises C₄ and C₅ hydrocarbons.

In a preferred embodiment, the C₃, C₄ and C₅ hydrocarbons are derived from C₃, C₄ and C₅ hydrocarbons separated from the product gas flow.

In a preferred embodiment, the C₄ and C₅ hydrocarbons are derived from C₄ and C₅ hydrocarbons separated from the product gas flow.

The C₃, C₄ and C₅ hydrocarbons refer to hydrocarbons with 3, 4 and 5 carbon atoms.

The C₄ and C₅ hydrocarbons refer to hydrocarbons with 4 and 5 carbon atoms.

In the embodiment described in the present application, the naphtha feedstock has a latent aromatic content of 0-80wt%, the single-pass conversion rate of naphtha is 70-95wt% and the single-pass conversion rate of methanol is about 100 wt%, the unconverted naphtha is separated from the product gas and returned to the naphtha and methanol coupled aromatization reactor 1 as a raw material, some light alkanes are separated from the product gas and returned to the riser reactor in the light hydrocarbon aromatization reactor 3 as a raw material, C₃, C₄ and C₅ hydrocarbons are separated from the product gas and returned to the bed reactor in the light hydrocarbon aromatization reactor 3 as a raw material, and the final product distribution is: 60-72wt% BTX, 8-15wt% light olefins, 3-7wt% hydrogen, 3-7wt% light alkanes, 4-6wt% combustible gas, 4-8wt% heavy aromatics, and 0.5-1wt% coke. The content of p-xylene in the mixed xylene in the product is 60-75 wt%.

The catalyst used in the following Examples was prepared as follows:
100 g of HZSM-5 zeolite molecular sieve (manufactured by Nankai University Catalyst Plant, Si/Al = 15) was immersed in a 10 wt% zinc nitrate aqueous solution. The mass ratio of HZSM-5 zeolite to the zinc nitrate solution (solid-to-liquid ratio) was 1:10. The mixture was impregnated at 80°C for 6 hours, filtered, dried at 120°C under air atmosphere for 4 hours, and then calcined at 550°C under air atmosphere for 4 hours to obtain a [Zn]HZSM-5 molecular sieve sample.
100 g of the [Zn]HZSM-5 molecular sieve sample was mixed with an amorphous binder containing aluminum or silicon. Detailed steps:
The [Zn]HZSM-5 sample, pseudo-boehmite, silica sol, xanthan gum (bio-gum), and water were uniformly mixed. The mixture underwent slurrying, homogenization, and deaeration to form a slurry. The weight fractions of the slurry components were:

| | |
|---|---|
| [Zn]HZSM-5 | 35 parts by weight |
| Al₂O₃ | 20 parts by weight |
| SiO₂ | 45 parts by weight |
| H₂O | 240 parts by weight |
| xanthan gum | 1 parts by weight |

The slurry was spray-dried to form microsphere particles with a particle size distribution of 20 to 100 µm. The particles were calcined in a muffle furnace at 550°C for 3 hours, yielding a [Zn]HZSM-5 formed molecular sieve with an attrition index of 1.2.

### Example 1

This embodiment employs the device illustrated in Fig. 1.

Naphtha feedstock fed to the naphtha and methanol coupled aromatization reactor is coal direct liquefaction naphtha with an aromatics potential content of 78 wt%.

Naphtha and methanol coupled aromatization reaction zone conditions: gas superficial velocity is 0.5 m/s, reaction temperature is 600°C, reaction pressure is 100 kPa, bed density is 700 kg/m³.

### Regeneration gas is air.

Regeneration zone conditions in the regenerator: gas superficial velocity is 0.5 m/s, regeneration temperature is 745°C, regeneration pressure is 100 kPa, bed density is 700 kg/m³.

Carbon content in the regenerated catalyst is 0.2 wt%.

Riser reactor feedstock is light alkanes separated from the product gas flow.

Riser reactor conditions: gas superficial velocity is 3.0 m/s, temperature is 690°C, pressure is 100 kPa, bed density is 150 kg/m³.

Bed reactor feedstock is unconverted naphtha separated from the product gas flow, with the main components of the unconverted naphtha being linear aliphatic hydrocarbons, branched aliphatic hydrocarbons and naphthenes of C₄-C₁₂.

Light hydrocarbon aromatization reaction zone conditions: gas superficial velocity is 0.5 m/s, reaction temperature is 665°C, reaction pressure is 100 kPa, bed density is 700 kg/m³.

Single-pass conversion of naphtha feedstock into the naphtha and methanol coupled aromatization reactor is 60 wt%.

Product distribution: 72 wt% BTX, 8 wt% light olefins, 3 wt% hydrogen, 3.5 wt% light alkanes, 5 wt% combustible gas, 7.5 wt% heavy aromatics, 1 wt% coke. The content of p-xylene in the mixed xylene in the product is 60 wt%.

### Example 2

This embodiment employs the device illustrated in Fig. 1.

Naphtha feedstock fed to the naphtha and methanol coupled aromatization reactor is coal direct liquefaction naphtha with an aromatics potential content of 0.1 wt%, including unconverted naphtha recycled from the separated product gas flow.

Naphtha and methanol coupled aromatization reaction zone conditions: gas superficial velocity is 2.0 m/s, reaction temperature is 510°C, reaction pressure is 500 kPa, bed density is 150 kg/m³.

Regeneration gas is oxygen.

Regeneration zone conditions in the regenerator: gas superficial velocity is 2.0 m/s, regeneration temperature is 610°C, regeneration pressure is 500 kPa, bed density is 150 kg/m³.

Carbon content in the regenerated catalyst is 0.1 wt%.

Riser reactor feedstock is light alkanes separated from the product gas flow and water vapor, with a water vapor content of 80 wt%.

Riser reactor conditions: gas superficial velocity is 10.0 m/s, temperature is 580°C, pressure is 500 kPa, bed density is 50 kg/m³.

Bed reactor feedstock is C₃, C₄, and C₅ hydrocarbons separated from the product gas flow.

Light hydrocarbon aromatization reaction zone conditions: gas superficial velocity is 2.0 m/s, reaction temperature is 550°C, reaction pressure is 500 kPa, bed density is 150 kg/m³.

Single-pass conversion of naphtha feedstock into the naphtha and methanol coupled aromatization reactor is 76 wt%.

Product distribution: 64 wt% BTX, 14 wt% light olefins, 5.3 wt% hydrogen, 3 wt% light alkanes, 5 wt% combustible gas, 8 wt% heavy aromatics, 0.7 wt% coke. The content of p-xylene in the mixed xylene in the product is 73 wt%.

### Example 3

This embodiment employs the device illustrated in Fig. 1.

Naphtha feedstock fed to the naphtha and methanol coupled aromatization reactor is coal direct liquefaction naphtha with an aromatics potential content of 3 wt%, including unconverted naphtha recycled from the separated product gas flow.

Naphtha and methanol coupled aromatization reaction zone conditions: gas superficial velocity is 1.2 m/s, reaction temperature is 550°C, reaction pressure is 120 kPa, bed density is 260 kg/m³.

Regeneration gas is oxygen-enriched air.

Regeneration zone conditions in the regenerator: gas superficial velocity is 1.2 m/s, regeneration temperature is 650°C, regeneration pressure is 120 kPa, bed density is 260 kg/m³.

Carbon content in the regenerated catalyst is 0.3 wt%.

Riser reactor feedstock is light alkanes separated from the product gas flow and water vapor, with a water vapor content of 25 wt%.

Riser reactor conditions: gas superficial velocity is 7.0 m/s, temperature is 630°C, pressure is 120 kPa, bed density is 80 kg/m³.

Bed reactor feedstock is C₄ and C₅ hydrocarbons separated from the product gas flow.

Light hydrocarbon aromatization reaction zone conditions: gas superficial velocity is 1.2 m/s, reaction temperature is 580°C, reaction pressure is 120 kPa, bed density is 260 kg/m³.

Single-pass conversion of naphtha feedstock into the naphtha and methanol coupled aromatization reactor is 80 wt%.

Product distribution: 60 wt% BTX, 15 wt% light olefins, 7 wt% hydrogen, 7 wt% light alkanes, 5 wt% combustible gas, 5.5 wt% heavy aromatics, 0.5 wt% coke. The content of p-xylene in the mixed xylene in the product is 75 wt%.

### Example 4

This embodiment employs the device illustrated in Fig. 1.

Naphtha feedstock fed to the naphtha and methanol coupled aromatization reactor is coal direct liquefaction naphtha with an aromatics potential content of 46 wt%, including unconverted naphtha recycled from the separated product gas flow.

Naphtha and methanol coupled aromatization reaction zone conditions: gas superficial velocity is 1.8 m/s, reaction temperature is 590°C, reaction pressure is 200 kPa, bed density is 220 kg/m³.

Regeneration gas is air.

Regeneration zone conditions in the regenerator: gas superficial velocity is 1.8 m/s, regeneration temperature is 700°C, regeneration pressure is 200 kPa, bed density is 220 kg/m³.

Carbon content in the regenerated catalyst is 0.1 wt%.

Riser reactor feedstock is light alkanes separated from the product gas flow and water vapor, with a water vapor content of 50 wt%.

Riser reactor conditions: gas superficial velocity is 5.0 m/s, temperature is 660°C, pressure is 220 kPa, bed density is 110 kg/m³.

Bed reactor feedstock is C₄ and C₅ hydrocarbons separated from the product gas flow.

Light hydrocarbon aromatization reaction zone conditions: gas superficial velocity is 1.8 m/s, reaction temperature is 630°C, reaction pressure is 200 kPa, bed density is 220 kg/m³.

Single-pass conversion of naphtha feedstock into the naphtha and methanol coupled aromatization reactor is 71 wt%.

Product distribution: 67 wt% BTX, 13 wt% light olefins, 6 wt% hydrogen, 5 wt% light alkanes, 4 wt% combustible gas, 4.2 wt% heavy aromatics, 0.8 wt% coke. The content of p-xylene in the mixed xylene in the product is 68 wt%.

### Example 5

This embodiment employs the device illustrated in Fig. 1.

Naphtha feedstock fed to the naphtha and methanol coupled aromatization reactor is coal direct liquefaction naphtha with an aromatics potential content of 64 wt%, including unconverted naphtha recycled from the separated product gas flow.

Naphtha and methanol coupled aromatization reaction zone conditions: gas superficial velocity is 1.0 m/s, reaction temperature is 580°C, reaction pressure is 150 kPa, bed density is 350 kg/m³.

Regeneration gas is air.

Regeneration zone conditions in the regenerator: gas superficial velocity is 1.0 m/s, regeneration temperature is 680°C, regeneration pressure is 150 kPa, bed density is 350 kg/m³.

Carbon content in the regenerated catalyst is 0.5 wt%.

Riser reactor feedstock is light alkanes separated from the product gas flow and water vapor, with a water vapor content of 40 wt%.

Riser reactor conditions: gas superficial velocity is 7.0 m/s, temperature is 650°C, pressure is 150 kPa, bed density is 80 kg/m³.

Bed reactor feedstock is C₄ and C₅ hydrocarbons separated from the product gas flow.

Light hydrocarbon aromatization reaction zone conditions: gas superficial velocity is 1.0 m/s, reaction temperature is 610°C, reaction pressure is 150 kPa, bed density is 350 kg/m³.

Single-pass conversion of naphtha feedstock into the naphtha and methanol coupled aromatization reactor is 67 wt%.

Product distribution: 70 wt% BTX, 10 wt% light olefins, 5 wt% hydrogen, 3 wt% light alkanes, 6 wt% combustible gas, 5 wt% heavy aromatics, 1.0 wt% coke. The content of p-xylene in the mixed xylene in the product is64 wt%.

The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A fluidized bed device for coupling naphtha and methanol to prepare aromatics and co-produce olefins, **characterized in that** the device comprises a naphtha and methanol coupled aromatization reactor, a regenerator, and a light hydrocarbon aromatization reactor;
the naphtha and methanol coupled aromatization reactor is connected to the regenerator via a spent catalyst delivery pipe I; the regenerator is connected to the naphtha and methanol coupled aromatization reactor via a regenerated catalyst delivery pipe;
a naphtha and methanol coupled aromatization reactor distributor is provided in the naphtha and methanol coupled aromatization reaction zone; the naphtha and methanol coupled aromatization reactor distributor comprises n sub-distributors, sequentially arranged from bottom to top as the 1^{st} sub-distributor to the n^{th} sub-distributor, where 2 ≤ n ≤ 10; the 1st sub-distributor is used to introduce naphtha feedstock; the 2^{nd} to n^{th} sub-distributors are used to introduce methanol feedstock;
the light hydrocarbon aromatization reactor comprises a riser reactor, and the riser reactor is connected to a bed reactor;
the regenerator is connected to the riser reactor via a regenerated catalyst slide valve II; the bed reactor is connected to the regenerator via a spent catalyst delivery pipe II.

2. The device for producing aromatics from naphtha according to claim 1, **characterized in that** the upper part of the naphtha and methanol coupled aromatization reactor is provided with a gas-solid separation zone; the gas-solid separation zone is provided with a product gas delivery pipe I; the lower part of the naphtha and methanol coupled aromatization reactor is provided with a naphtha and methanol coupled aromatization reaction zone;
the naphtha and methanol coupled aromatization reactor shell is further provided with a gas-solid separation unit I, a gas-solid separation unit II, and a gas collection chamber I; the gas collection chamber I is located at the top of the gas-solid separation zone, and the gas collection chamber I is connected to the product gas delivery pipe I; the gas outlets of the gas-solid separation unit I and the gas-solid separation unit II are connected to the gas collection chamber I;
the catalyst outlet end of the gas-solid separation unit I is located above the opening end of the stripper I inlet pipe; the inlet of the gas-solid separation unit II is connected to the regenerator; the catalyst outlet end of the gas-solid separation unit II is located above the opening end of the stripper I inlet pipe and between the 1^{st} and 2^{nd} sub-distributors.

3. The device for producing aromatics from naphtha according to claim 2, **characterized in that** a stripper I is provided below the naphtha and methanol coupled aromatization reaction zone; the naphtha and methanol coupled aromatization reaction zone is connected to the spent catalyst delivery pipe I via the stripper I.

4. The device for producing aromatics from naphtha according to claim 3, **characterized in that** the stripper I is connected to the spent catalyst delivery pipe I via a spent catalyst slide valve I.

5. The device for producing aromatics from naphtha according to claim 2, **characterized in that** the gas-solid separation unit I employs one or more sets of gas-solid cyclone separators, each set comprising a first-stage gas-solid cyclone separator and a second-stage gas-solid cyclone separator.

6. The device for producing aromatics from naphtha according to claim 2, **characterized in that** the gas-solid separation unit II employs one or more sets of gas-solid cyclone separators, each set comprising a first-stage gas-solid cyclone separator and a second-stage gas-solid cyclone separator.

7. The device according to claim 1, **characterized in that** the upper part of the regenerator is provided with a regenerator gas-solid separation zone; the regenerator gas-solid separation zone is provided with a flue gas delivery pipe;
the lower part of the regenerator is provided with a regeneration zone; the spent catalyst delivery pipe I outlet and the spent catalyst delivery pipe II deliver a spent catalyst into the regeneration zone; the lower part of the regenerator is provided with a regenerator distributor for introducing a regeneration gas;
the regenerated catalyst delivery pipe transports the regenerated catalyst from the regeneration zone to the naphtha and methanol coupled aromatization reactor; the regenerated catalyst slide valve II delivers the regenerated catalyst to the riser reactor.

8. The device for producing aromatics from naphtha according to claim 7, **characterized in that** the regenerator shell is further provided with a regenerator gas-solid separation unit and a regenerator gas collection chamber; the regenerator gas collection chamber is located at the top of the regenerator gas-solid separation zone; the gas outlet of the regenerator gas-solid separation unit is connected to the regenerator gas collection chamber; the regenerator gas collection chamber is connected to the flue gas delivery pipe.

9. The device for producing aromatics from naphtha according to claim 7, **characterized in that** a regenerator stripper is provided below the regeneration zone; the regeneration zone is connected to the regenerated catalyst slide valve I and the regenerated catalyst slide valve II via the regenerator stripper.

10. The device for producing aromatics from naphtha according to claim 9, **characterized in that** the regenerated catalyst slide valve I is connected to the naphtha-to-aromatics reactor via the regenerated catalyst delivery pipe; the regenerated catalyst slide valve II is connected to the riser reactor.

11. The device for producing aromatics from naphtha according to claim 7, **characterized in that** the regenerator gas-solid separation unit employs one or more sets of gas-solid cyclone separators, each set comprising a first-stage gas-solid cyclone separator and a second-stage gas-solid cyclone separator.

12. The device according to claim 1, **characterized in that** the upper part of the bed reactor is provided with a bed reactor gas-solid separation zone; the bed reactor gas-solid separation zone is provided with a product gas delivery pipe II;
the lower part of the bed reactor is provided with a light hydrocarbon aromatization reaction zone; the lower inner part of the light hydrocarbon aromatization reaction zone is provided with a bed reactor distributor; the bed reactor distributor is used to introduce a bed reactor feedstock; the upper end of the riser reactor penetrates the bottom of the bed reactor and is axially inserted into the bed reactor;
the regenerated catalyst slide valve II delivers the catalyst to the feedstock inlet end of the riser reactor.

13. The device according to claim 12, **characterized in that** the gas-solid separation zone of the bed reactor is provided with a gas-solid separation unit III and a gas collection chamber II; the gas outlet of the gas-solid separation unit III is connected to the gas collection chamber II; the catalyst outlet of the gas-solid separation unit III is located in the light hydrocarbon aromatization reaction zone; the gas collection chamber II is connected to the product gas delivery pipe II located outside the bed reactor.

14. The device according to claim 13, **characterized in that** the light hydrocarbon aromatization reaction zone is connected to a stripper II, and the bed reactor is connected to the spent catalyst delivery pipe II via the stripper II.

15. The device according to claim 14, **characterized in that** the stripper II is connected to the spent catalyst delivery pipe II via a spent catalyst slide valve II.

16. The device according to claim 13, **characterized in that** the gas-solid separation unit III is a gas-solid cyclone separator; the catalyst outlet of the gas-solid separation unit III is located above the outlet end of the riser reactor.

17. A method for coupling naphtha and methanol to prepare aromatics and co-produce olefins, **characterized in that** the device according to any one of claims 1 to 16 is used; the catalyst is a metal molecular sieve bifunctional catalyst;
a feedstock containing naphtha is introduced into the naphtha and methanol coupled aromatization reactor via the 1^{st} sub-distributor; a feedstock containing methanol is introduced into the naphtha and methanol coupled aromatization reactor via the 2^{nd} to n^{th} sub-distributors; a riser reactor feedstock containing light alkanes is introduced into the riser reactor; a bed reactor feedstock is introduced into the bed reactor; a regeneration gas is introduced into the regenerator;
the naphtha and methanol coupled aromatization reactor and the bed reactor output a product gas, and a spent catalyst is delivered to the regenerator via the spent catalyst delivery pipe I and the spent catalyst delivery pipe II; after the spent catalyst is regenerated by reacting with regeneration gas in the regenerator, it is delivered to the naphtha and methanol coupled aromatization reactor and the riser reactor; the regenerator discharges a flue gas.

18. The method according to claim 17, **characterized in that** the catalyst is a metal-modified HZSM-5 zeolite molecular sieve; a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

19. The method according to claim 17, **characterized in that** the naphtha is at least one selected from the group consisting of coal direct liquefaction naphtha, coal indirect liquefaction naphtha, straight-run naphtha, and hydrocracking naphtha.

20. The method according to claim 17, **characterized in that** the feedstock containing naphtha further comprises unconverted naphtha separated from the product gas flow, and the unconverted naphtha comprises linear aliphatic hydrocarbons, branched aliphatic hydrocarbons, and naphthenes of C₄-C₁₂.

21. The method according to claim 17, **characterized in that** the reaction conditions of the naphtha and methanol coupled aromatization reaction zone are: gas superficial linear velocity of 0.5-2.0 m/s, reaction temperature of 500-600°C, reaction pressure of 100-500 kPa, and bed density of 150-700 kg/m³.

22. The method according to claim 17, **characterized in that** the carbon content in the regenerated catalyst is ≤0.5 wt%.

23. The method according to claim 17, **characterized in that** the regeneration gas is at least one selected from the group consisting of oxygen, air, and oxygen-enriched air.

24. The method according to claim 17, **characterized in that** the reaction conditions of the regeneration zone are: gas superficial linear velocity of 0.5-2.0 m/s, regeneration temperature of 600-750°C, regeneration pressure of 100-500 kPa, and bed density of 150-700 kg/m³.

25. The method according to claim 17, **characterized in that** the riser reactor feedstock further comprises water vapor, with a water vapor content of 0-80 wt%.

26. The method according to claim 17, **characterized in that** the light alkanes in the riser reactor feedstock are obtained by separation from the product gas flow.

27. The method according to claim 17, **characterized in that** the reaction conditions of the riser reactor are: gas superficial linear velocity of 3.0-10.0 m/s, temperature of 580-700°C, pressure of 100-500 kPa, and bed density of 50-150 kg/m³.

28. The method according to claim 17, **characterized in that** the bed reactor feedstock is obtained by separation from the product gas flow.

29. The method according to claim 17, **characterized in that** the bed reactor feedstock comprises unconverted naphtha separated from the product gas flow, and the unconverted naphtha comprises linear aliphatic hydrocarbons, branched aliphatic hydrocarbons, and naphthenes of C₄-C₁₂.

30. The method according to claim 17, **characterized in that** the bed reactor feedstock comprises C₃, C₄, and C₅ hydrocarbons.

31. The method according to claim 17, **characterized in that** the bed reactor feedstock comprises C₄ and C₅ hydrocarbons.

32. The method according to claim 17, **characterized in that** the reaction conditions of the light hydrocarbon aromatization reaction zone are: gas superficial linear velocity of 0.5-2.0 m/s, reaction temperature of 550-665°C, reaction pressure of 100-500 kPa, and bed density of 150-700 kg/m³.

33. The method according to claim 17, **characterized in that** naphtha enters the naphtha and methanol coupled aromatization reaction zone via the 1^{st} sub-distributor of the naphtha and methanol coupled aromatization reactor distributor, contacts the catalyst from the regenerator, and generates a product gas flow containing BTX, light olefins, hydrogen, light alkanes, combustible gas, heavy aromatics, and unconverted naphtha; the catalyst from the regenerator enters the gas-solid separation unit II to achieve gas-solid separation; a degassed catalyst enters the space between the 1^{st} and 2^{nd} sub-distributors; methanol enters the naphtha and methanol coupled aromatization reaction zone via the 2^{nd} to n^{th} sub-distributors of the naphtha and methanol coupled aromatization reactor distributor, reacts with benzene and toluene in the product gas flow to undergo methylation, generating para-xylene; the catalyst from the regenerator becomes spent catalyst due to coking in the naphtha and methanol coupled aromatization reaction zone; the product gas flow enters the gas-solid separation unit I to remove the spent catalyst entrained in it, then enters the gas collection chamber I, and is delivered to downstream sections via the product gas delivery pipe I; the spent catalyst in the naphtha and methanol coupled aromatization reaction zone enters the stripper I via the opening end of the stripper I inlet pipe, undergoes stripping, and after stripping, passes through the spent catalyst slide valve I and the spent catalyst delivery pipe I to enter downstream sections;
the regeneration gas is introduced into the regeneration zone of the regenerator via the regenerator distributor, contacts the spent catalyst from the naphtha and methanol coupled aromatization reactor and the spent catalyst from the light hydrocarbon aromatization reactor, and the coke on the spent catalyst reacts with the regeneration gas to generate the flue gas, converting the spent catalyst into the regenerated catalyst;
the riser reactor feedstock is introduced into the riser reactor via the inlet end of the riser reactor, contacts and reacts with the regenerated catalyst from the regenerator, and the riser reactor feedstock is converted into a flow containing BTX, light olefins, H₂, and other components under the action of the catalyst, then enters the lower inner part of the light hydrocarbon aromatization reaction zone in the bed reactor via the outlet end of the riser reactor;
the bed reactor feedstock is introduced into the light hydrocarbon aromatization reaction zone via the bed reactor distributor, contacts the catalyst from the riser reactor, and generates a light hydrocarbon aromatization product gas containing BTX, light olefins, H2, and other components, while the catalyst becomes the spent catalyst.
